# EUROPEAN PATENT APPLICATION

(11) **EP 2 454 993 A1**
(43) Date of publication of application: **23.05.2012**
(21) Application number: 11189443.2
(22) Date of filing: 16.11.2011
(51) Int. Cl.: A61B 6/00, G01V 5/00, G06F 19/00

(54) **System and method for performing a comprehensive health assessment**

(30) Priority: 23.11.2010 US 416637 P; 29.06.2011 US 171590
(71) Applicant: General Electric Company, Schenectady, NY 12345 (US)
(72) Inventor: Sabol, John Michael, Waukesha, Wisconsin 53188 (US); Murphy, Lawrence E., Waukesha, Wisconsin 53188 (US); Jabri, Kadri Nizar, Waukesha, Wisconsin 53188 (US); Lingamneni, Anila, Waukesha, Wisconsin 53188 (US); Robinson, Scott William, Waukesha, Wisconsin 53188 (US); Widmann, David L., Waukesha, Wisconsin 53188 (US)
(74) Representative: Bedford, Grant Richard

(57) **Abstract**

A system and method for providing a comprehensive overall health assessment is disclosed. A health assessment system (10) includes first and second support structures (14, 16) oriented in a vertical fashion and spaced apart from one another to define a scanning area (18) configured to receive a subject to be scanned, an x-ray imaging system (34, 36) incorporated into the first and second support structures (14, 16) and configured to acquire x-ray image data from the subject in a standing position, and at least one additional data acquisition device (28, 47, 48, 50, 52, 54) configured to acquire health related data from the subject. The health assessment system (10) also includes a computer (42) programmed to receive the x-ray image data and the health related data and generate a diagnostic output based on the x-ray image data and the health related data, the diagnostic output comprising at least one of a health related diagnosis and a recommendation for future action.

## Description

Various embodiments of the invention relate generally to diagnostic imaging and, more particularly, to a walk-through type imaging device configured to acquire multiple types of physiological and morphological data from a subject and that provides for a comprehensive assessment of the subject's overall health.

Walk-through imaging devices for acquiring information about a subject have become an increasingly valuable tool in recent years, as such devices enable high-throughput, efficient imaging of a large number of subjects in a very cost effective manner. One example of the implementation of such walk-through imaging devices is their use as airport and anti-theft scanners. It is known, however, that such walk-through imaging devices are limited in the data that they acquire, in that they are designed only for the specific purpose of security or theft prevention. Thus, the purpose of these walk-through imaging devices is not to diagnose or provide health information to the individual, and such devices, in fact, lack that capability to provide health information to/about the individual.

Similarly, there are walk-through devices and technology to screen individuals for specific physiological conditions such as body thermal imaging temperature monitors used at body control points for detection of individuals with elevated body temperatures indicative of potential carriers of infection. Again, however, the purpose of these devices is not to diagnose or provide health information to the individual, nor do they combine automatic integration of multiple types of data (patient history, electronic medical records (EMR) information, etc.). A critical difference is the lack of personalized metrics that the individual's health will be referenced against. Instead, the prior art devices described above reference the measured parameter from the individual against a population norm.

Thus, existing walk-through devices thus fail to enable and facilitate a "one-stop" examination where a single walk-through device acquires and obtains a range of diagnostic test information. Such a one-stop examination device would be beneficial in that this would increase efficiency and could lower overall cost to a health care enterprise. Furthermore, such a one-stop examination device could be implemented to provide a comprehensive assessment of the patient's overall health in "non-traditional" locations where obtaining a complete patient diagnosis is difficult. That is, one-stop examination devices could be situated in areas where there is insufficient provider coverage (e.g., rural areas or developing countries), where more convenience is desired (e.g., shopping malls, drugstores), or where independent, rapid health screens are required (e.g., natural disasters, military deployments, border entry points), for example.

Therefore, it would be desirable to design a walk-through type imaging device capable of acquiring patient data in a "one-stop" type examination and that provides for diagnosing or providing health information to/about an individual. It would also be desirable for such a walk-through type imaging device to combine automatic integration of multiple types of patient data to provide a comprehensive assessment of the patient's overall health.

Various embodiments of the invention are directed to a method and apparatus for acquiring multiple types of physiological and morphological data from a subject and providing for a comprehensive assessment of the subject's overall health. A walk-through type imaging device is provided that enables the acquisition of patient data in a "one-stop" type examination and the diagnosing or providing of health information to or about an individual.

According to an aspect of the invention, a health assessment system includes a first support structure oriented in a vertical fashion, a second support structure oriented in a vertical fashion and spaced apart from the first support structure to define a scanning area configured to receive a subject to be scanned, an x-ray imaging system incorporated into the first and second support structures and configured to acquire x-ray image data from the subject in a standing position, and at least one additional data acquisition device configured to acquire health related data from the subject. The health assessment system also includes a computer programmed to receive the x-ray image data and the health related data and generate a diagnostic output based on the x-ray image data and the health related data, the diagnostic output comprising at least one of a health related diagnosis and a recommendation for future action.

According to another aspect of the invention, a walk-through health assessment system includes a gate structure having a first vertical member and a second vertical member spaced apart from the first vertical member to define a scanning area, an x-ray imaging system secured to the gate structure and configured to acquire x-ray image data from a subject when standing in the scanning area, and a means for acquiring additional health related data from the subject separate from the x-ray image data. The walk-through health assessment system also includes a computer programmed to receive the x-ray image data and the additional health related data, extract diagnostic information from the x-ray image data and the health related data, and generate a subject health assessment from the extracted diagnostic information.

According to yet another aspect of the invention, a method of acquiring physiological and biological data on a subject by way of a walk-through health assessment system includes positioning a subject within an scanning area of a gate structure of the walk-through health assessment system, with the gate structure including a first vertical member and a second vertical member spaced apart from the first vertical to define a scanning area. The method also includes acquiring x-ray image data from the subject by way of a linear x-ray source and x-ray detector arrangement and acquiring additional health related data from the subject by way of at least one additional data acquisition device. The method further includes extracting diagnostic information from the x-ray image data and the additional health related data and generating a subject health assessment from the extracted diagnostic information.

These and other advantages and features will be more readily understood from the following detailed description of preferred embodiments of the invention that is provided in connection with the accompanying drawings.

The drawings illustrate preferred embodiments presently contemplated for carrying out the invention.

In the drawings:
FIGS. 1 and 2 are perspective views of a walk-through health assessment system according to an embodiment of the invention.
FIG. 3 is a schematic diagram of the walk-through health assessment system of FIGS. 1 and 2 according to an embodiment of the invention.
FIGS. 4-9 illustrate clinical examples of conditions and/or diseases diagnosable by way of a subject examination in the health assessment system of FIGS. 1 and 2, according to embodiments of the invention.

According to certain embodiments of the invention, a health assessment system is provided that provides for quick and efficient acquisition and processing of physiological and/or anatomical data from a subject. The health assessment system is configured as a "one-stop" examination where a single system acquires and obtains a range of diagnostic test information. For acquiring such data, health assessment system includes a plurality of data capture devices configured to acquire or receive image data as well as physiological and health related data from the subject. For processing the acquired/received subject data, diagnostic devices are provided in the health assessment system that enable a health related diagnosis for the subject or recommendation for further action, for example. Health assessment system thus enables a more complete assessment of the subject's overall health than that provided by a system or device that captures just one physiological or biological related parameter from a subject, such as a stand-alone x-ray examination or imaging system, for example.

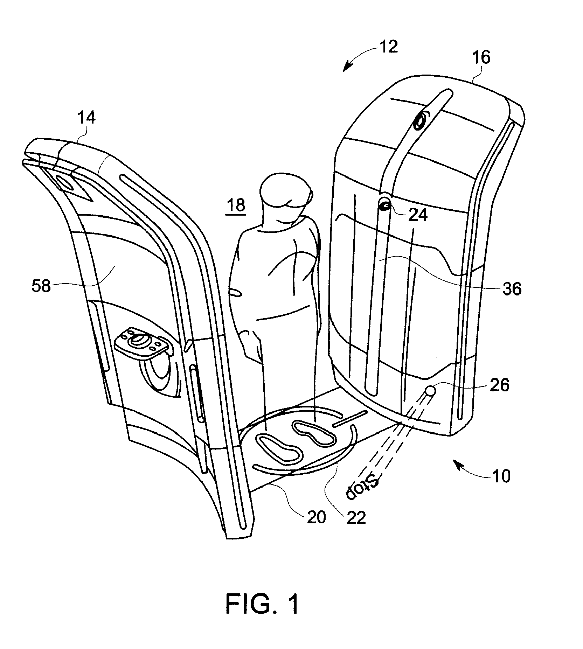

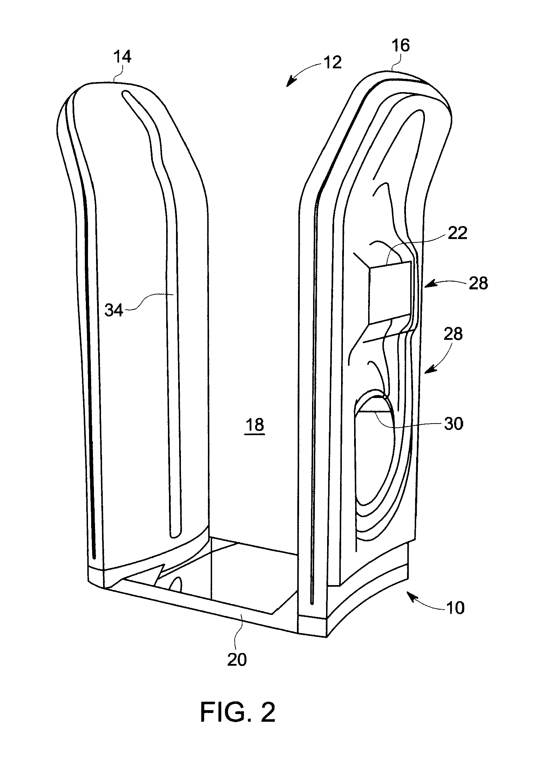

Referring to FIGS. 1-3, perspective and schematic views of a health assessment system 10 are shown according to one embodiment of the invention. As shown in FIGS. 1 and 2, the health assessment system 10 is configured as a walk-through health assessment system that implements a 'gate' design that enables a subject to walk or pass through the device without mechanical assistance, so as to provide for quick and efficient acquisition of physiological and/or anatomical data from the subject. The walk-through health assessment system 10 includes a gate structure 12 having a first support structure 14 and a second support structure 16 each oriented in an upright or vertical manner. The first and second support structures 14, 16 are spaced apart so as to define an imaging volume (i.e., scanning area) 18 of the imaging system 10. The first and second support structures 14, 16 are thus spaced apart a distance sufficient to accommodate, within the imaging area 18, subjects of a variety of shapes and sizes, encompassing a majority of the population. A base structure 20 extends between the first and second support structures 14, 16 and is connected to the support structures at a footing or bottom edge thereof. The base structure 20 can include a marked-off or highlighted region 22 thereon that indicates a desired location or locations for a subject to stand on for performing an x-ray image acquisition. According to one embodiment, a weight sensor or positioning/proximity sensor (not shown) is included in base structure 20 to determine when the subject is properly positioned for initiation of the x-ray image acquisition.

Subject guidance mechanisms are integrated into health assessment system 10 and provide instructions and/or prompts to a subject regarding operation of the system, such that the subject can operate the system independently and without the assistance of trained medical personnel. The subject may follow written, audible or visual cues provided by subject guidance mechanism to facilitate the acquisition of a range of diagnostic test information and enable a health related diagnosis for the subject and/or recommendation for further action. As shown in FIGS. 1 and 2, according to one embodiment, a subject guidance mechanism is provided in the form of an indicator light 24 in the gate structure 12 that regulates the flow of subjects through the health assessment system 10, such as by being illuminated in green to indicate that a subject should enter the scanning area 18 and/or by being illuminated in red to indicate that scanning of a subject is in process. Additionally, a projection device 26 is provided that functions to provide visual instructions to the subject regarding the health assessment scan. For example, the projection device 26 may illuminate onto the ground instructions to the subject as to how to position himself/herself in the scanning area 18, such as by generating a "Stop" instruction or "Step Forward/Backward" instruction.

Another subject guidance mechanism is provided in health assessment system 10 in the form of a user interface 28 with which the subject interacts. The user interface 28 is located on one of the first or second support structures 14, 16, on either an inward or outward facing surface thereof so as to provide easy access for the subject. The user interface 28 is configured to serve several functions for the acquisition of health data from a subject -- including gathering information identifying the subject and gathering health related information/data on the subject.

As indicated, the user interface 28 assists in gathering identifying information from the subject to be examined, with the user interface 28 allowing for the subject to identify himself/herself in several ways. According to one embodiment, user interface 28 includes a device reader 30 configured to receive/accommodate a data storage and transport device which the subject possesses. For example, as shown in FIG. 2, device reader 30 may be in the form of a card reader 30 through which subject could swipe a "health card" upon arrival at system, such as a magnetic card reader, RF card reader, or similar device. Alternatively, device reader may be in the form of a USB port (not shown) into which the subject could plug-in a flash drive or similar device. The health card or flash drive would contain data identifying the subject to be examined. According to another embodiment, the user interface 28 also includes a data input device 32 through which subject can input identification information. Data input device 32 can be configured to receive verbal or manual input from subject regarding identification information. Thus, for example, subject may input via a keyboard or touch screen 32, a biometric identification number to which subject information is linked/associated.

According to another embodiment, user interface 28 is configured to gather identifying information from the subject by way of facial recognition technology. That is, a video capture device (not shown) is integrated into user interface 28 that works in conjunction with facial recognition software programmed in user interface 28 in order to capture a facial image from the subject and process the image for identification purposes. The implementation of facial recognition capability into user interface 28 can thus provide for verification of the identity of the subject, so as to enhance user safety by providing protection from identity theft, such as by theft of a subject health card, flash drive, or similar health data storage device.

In functioning as a "one-stop" examination system, the health assessment system 10 acquires and integrates multiple types of patient data so as to provide a comprehensive assessment of the subject's overall health. For acquiring such data, health assessment system 10 includes a plurality of data capture devices configured to acquire or receive physiological and health related data from the subject.

One such type of data acquired from the subject by health assessment system 10 is x-ray image data. To provide for such x-ray image data acquisition, an x-ray source 34 and a detector assembly or arrangement 36 are incorporated into the first and second support structures 14, 16, respectively. As shown in FIGS. 1 and 2, according to one embodiment of the invention, the x-ray source 34 and detector assembly 36 are configured as a linear x-ray source and linear detector assembly that are oriented in a vertical direction, with the linear x-ray source 34 and the detector assembly 36 aligned with one another on opposite sides of the walk-through health assessment system 10. Thus, the x-ray source 34 may be formed as: a distributed source that includes a plurality of x-ray sources, such as field emitter devices, or other controllable and addressable x-ray sources, that are linearly aligned in the vertical direction; a single, stationary x-ray source; or a moveable x-ray source designed to slide in the vertical direction within first support structure 14. The detector assembly 36 may be generally similar to conventional digital x-ray detectors, including a scintillator for converting x-ray radiation to lower energy photons, photodiodes for receiving the photons from the scintillator, and transistors for reading out the charge depletion in the photodiodes resulting from the interacting photons. Alternatively, according to an embodiment of the invention, the detector assembly 36 may include a direct conversion material (i.e., converts x-rays directly to electrical signals) and be configured as a photon counting device, so as to enable energy discrimination or imaging with multiple energy bands. The detector assembly 36 may have the format of a rectangular area of sufficient size to acquire a view of the entire anatomy of interest in a single acquisition, or the detector assembly 36 may be of smaller area and translate or rotate to acquire a complete image. While specific examples are provided above for the structure of x-ray source 34 and detector assembly 36, it is recognized that other forms/types of x-ray sources and detectors can be implemented in health assessment system 10, and thus the specific examples set forth above are not meant to limit the scope of the invention.

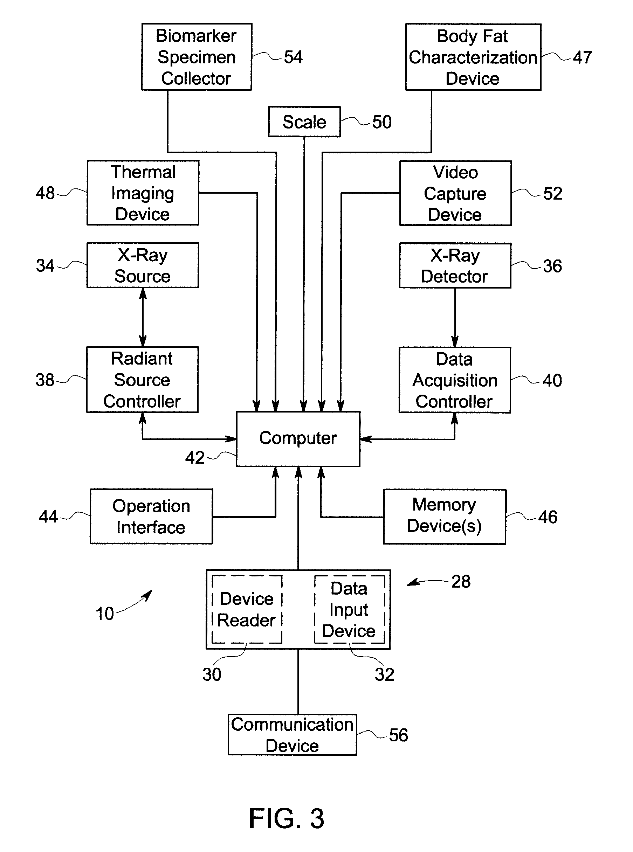

As shown in FIG. 3, to control operation of linear x-ray source 34 and detector arrangement 36, the health assessment system 10 includes a radiation source controller 38 and a data acquisition system (DAS) 40, which may both be in operative communication with a computer 42. The computer 42 may thus cause radiation source controller 38 to trigger emission of x-ray radiation, as well as receive data acquired by DAS 40 and coordinate storage and processing of that data. An operator interface 44 may be integral with the computer 42 and will generally include an operator workstation for initiating imaging sequences, controlling such sequences, and manipulating data acquired during imaging sequences, which can be stored in a memory device 46. Additionally, computer 42 may perform high speed reconstruction of the x-ray data from DAS 40 to generate an image of the subject or a particular region-of-interest (ROI) of the subject.

According to an exemplary embodiment of the invention, the linear x-ray source 34 and detector assembly 36 are configured so as to provide for x-ray image data acquisition that enables production of a tomographic image. That is, the linear x-ray source 34 is controlled to emit x-rays towards the subject from a plurality of positions relative to the linear detector arrangement 36, such that multiple projection images are acquired at differing angles based on the emission of x-rays from the linear x-ray source 34 at the plurality of positions. Based on the multiple projection images, a tomographic image is generated of at least a portion of the subject. Furthermore, according to an embodiment of the invention, the detector assembly 36 is formed from a direct conversion material (e.g., CZT) and configured as an energy sensitive detector capable of providing data or feedback as to the number and/or energy of photons detected. Data as to the number and/or energy of photons detected by detector assembly allows for multi-spectral x-ray image data to be acquired, with the linear x-ray source 34 performing a dual energy x-ray scan to provide for the capture of such data.

As shown in FIGS. 1-3, a plurality of additional data acquisition devices and/or sensors configured to acquire or receive physiological and health related data from the subject are also included in health assessment system 10, so as to enable a more complete assessment of the patient's overall health than provided by just an x-ray examination or image. For example, sensors and devices can be incorporated into health assessment system 10 that provide information such as basal temperature, bone density, height, weight, body mass index (BMI), derived metabolic health information, and additional physiological parameters. According to embodiments of the invention, the health assessment system 10 can include data acquisition devices and/or sensors configured to acquire medical diagnostic data from the patient in both a passive and active fashion. Data such as body fat percentage, body temperature, weight, and height could be assessed via passive type devices, including an electrical impedance percent body fat characterization device 47, a body thermal imaging device 48, a weight measurement device (e.g., scale) 50, and a video capture device 52, for example. An active device in the form of a biomarker specimen collector 54 could be integrated into health assessment system 10 to obtain a sample for analysis of biomarkers. For example, a "mouthpiece" could be provided into which the patient could be prompted to exhale in order to acquire exhaled breath or sputum. As another example, a blood glucose meter could be provided for which patient provides a blood sample, so as to aid in the tracking/diagnosis of diabetes.

As another means for acquiring physiological and health related data, the user interface 28 functions as a data acquisition device. That is, as set forth above, the user interface 28 is configured not only to provide for receipt of identification information on the subject, but is also configured to provide for the receipt or acquisition of health related data on the subject to be examined. According to an embodiment of the invention, the user interface 28 includes a device reader 30 configured to receive/accommodate a data storage and transport device which the subject possesses, with the device reader 30 being in the form of a card reader through which subject could swipe a "health card" upon arrival at system or, alternatively, a USB port into which the subject could plug is a flash drive or similar device. The health card or flash drive would contain data regarding the electronic medical records (EMR) of the subject that identifies/provides data regarding patient specific information and/or characteristics of the subject. The specific information provided by the health card/flash drive may include information on the user's past health history (i.e., illnesses, surgeries, chronic disease), family history, a review of current symptoms and/or health-related behaviors, and current medications and supplements, for example. The information may also include current measurement of various physiological parameters. Thus, for example, data regarding the user's current weight, height, and blood pressure, may be provided to system by way of the subject's interaction with user interface 28 (e.g., swiping the health card in device reader 30).

In an embodiment where a data input device 32 (e.g., keyboard, touch screen, etc.) is provided on user interface 28, the subject can input health related data by way of the device 32. Thus, for example, subject may input a textual description of a specific physiological or biological condition, numerical values for specific physiological or biological parameters, or select a health related description/condition from a menu, for example. According to one embodiment, where the subject inputs a biometric identification number, the user interface 28 links to the subject's EMR based on the provided biometric identification number. In such an embodiment, health assessment system 10 may include a communications device 56 that enables remote access to the patient's EMR, such as a wired or wireless link to a healthcare records storage facility or other record storage. Thus, for example, health assessment system 10 can include a transceiver 56 that may function in accordance with any desired wireless transition protocols, such as Blue tooth, infrared protocols, IEEE 802.11, and so forth, that provides for bi-directional communication of data back and forth from the patient's EMR.

In addition to the data acquisition devices and/or sensors that acquire or receive physiological and health related data from the subject, health assessment system 10 also includes diagnostic tools configured to process the acquired/received subject data, so as to enable a health related diagnosis for the subject or recommendation for further action, for example. Accordingly, the computer 42 of health assessment system 10 is programmed to process the physiological and biological data acquired/received on the subject by way of the data acquisition devices and/or sensors, such as the x-ray imaging data acquired by the x-ray source and detector combination 34, 36 and the health related data received by swiping of a health card through card reader 30. Algorithms and/or programming on the computer 42 enable automatic extraction of diagnostic information from the acquired data. For example, algorithms on the computer 42 can extract information from acquired x-ray image data, such as lung cancer detection via a computer aided diagnosis (CAD). Additionally, algorithms on the computer 42 provide for integration of the additional health related data, acquired via additional data acquisition devices 30, 32 and/or sensors 48-54, with the imaging data to obtain a health related diagnosis or a recommendation for further action. For example, CAD detection of suspicious patterns from a chest x-ray with body temperature and patient oral history could be used to diagnose pneumonia or differentiate an acute viral respiratory infection (common cold) from presentation of a potential pandemic infection such as SARS or H1N1 or TB and recommend further medical action or non-medical action such as border or facility access restriction.

According to various embodiments of the invention, the diagnostic output of health assessment system 10 is provided at the system to the subject and/or an operator of the system, or alternatively is transmitted to a remote location for further analysis. According to one embodiment, the diagnostic output of health assessment system 10, in the form of a health related diagnosis and/or recommendation for future action, is displayed on a display screen 58 (FIG. 1) to enable viewing by the subject and/or an operator of the system. According to another embodiment, the diagnostic output of health assessment system 10 is provided to subject in the form of a print-out. Beneficially, the diagnostic output is thus immediately viewable by the subject. According to another embodiment, the diagnostic output of health assessment system 10 is transmitted to a remote location, such as by way of communications device 56 (FIG. 3). For example, the diagnostic output of health assessment system 10 may be transmitted to trained health personnel, such that the health personnel have access to the health related diagnosis and/or recommendation output by computer 42 of health assessment system 10 and can perform further analysis thereon. The health personnel may then provide feedback to the subject, such as providing: instructions, feedback and help, guidance regarding additional on-site imaging, enhanced information gathering, or a referral for additional care. As another example, the diagnostic output of health assessment system 10 may be emailed to the subject, with such an email including health tips and a health risk assessment.

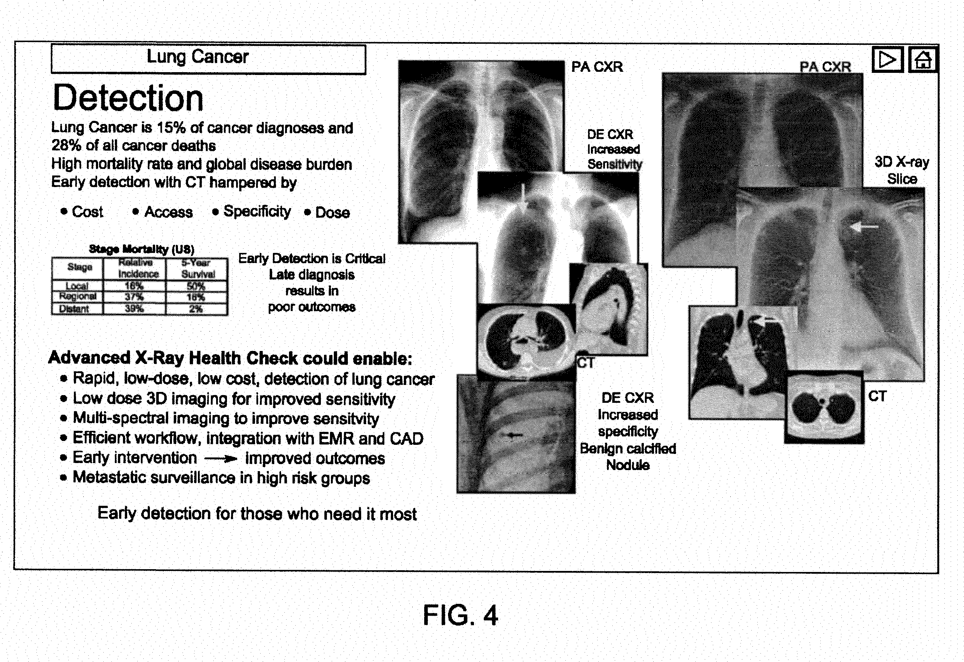

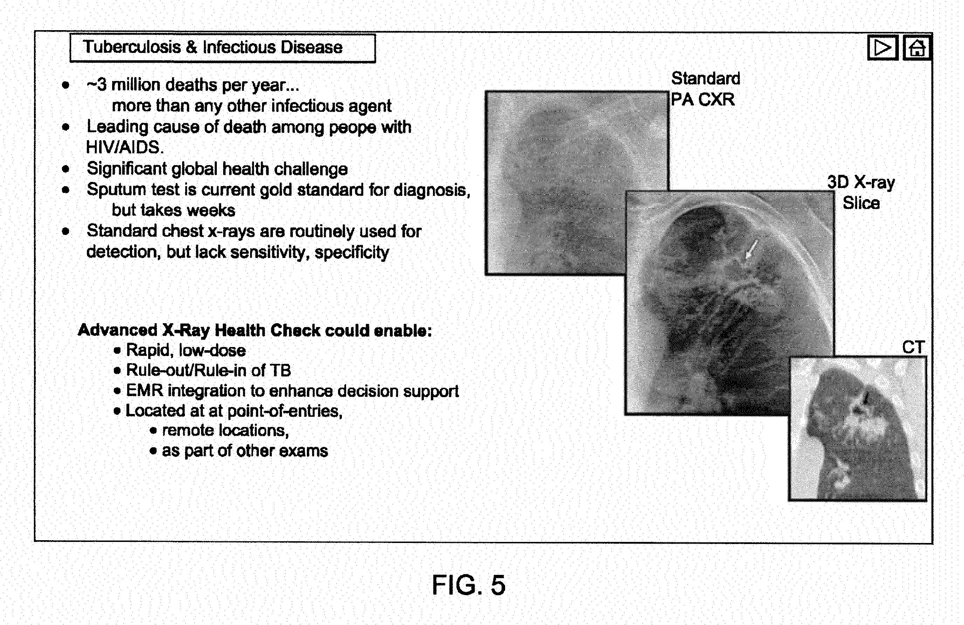

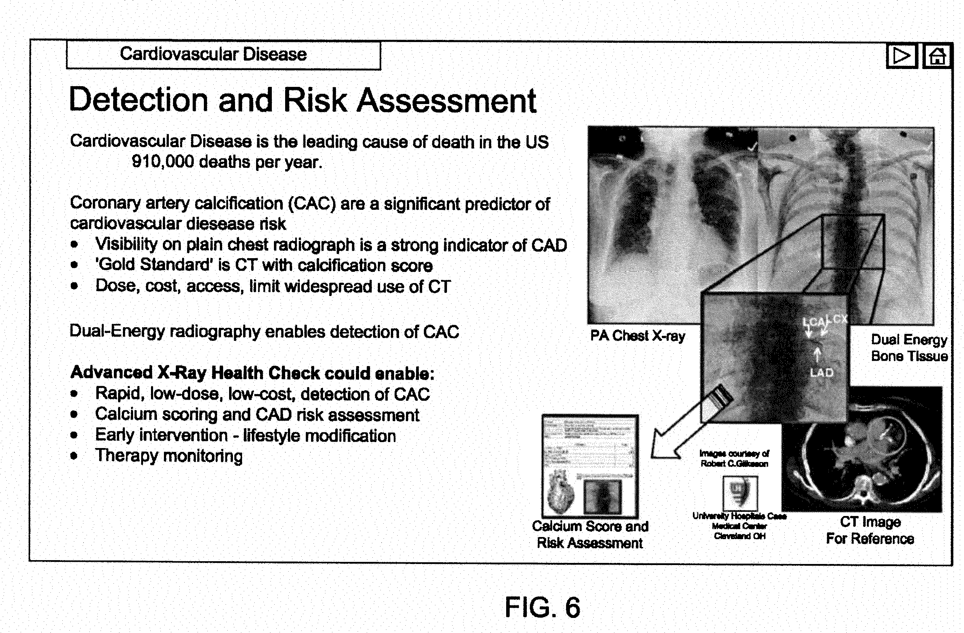

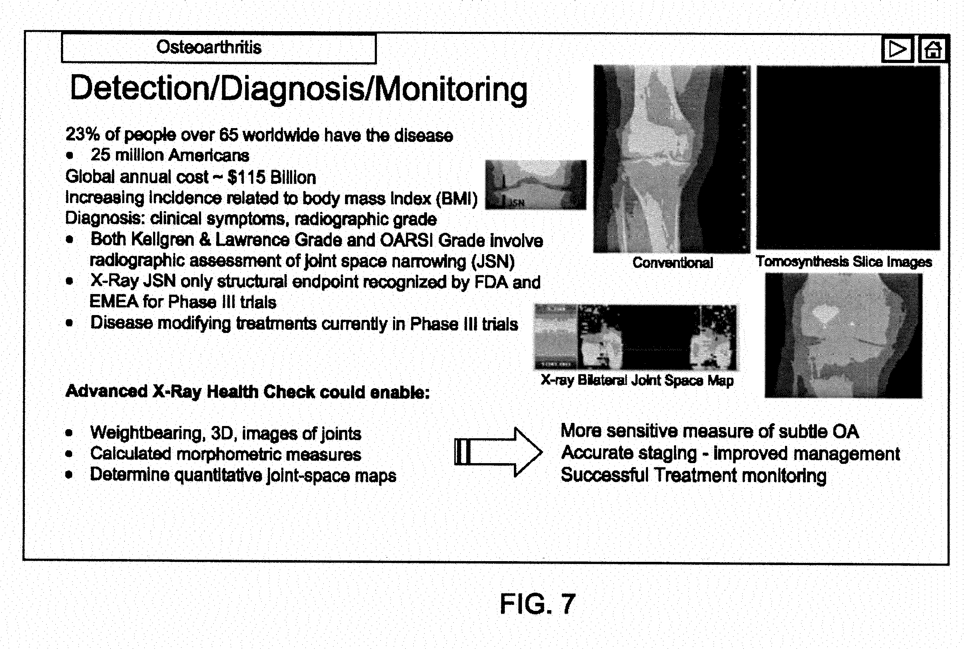

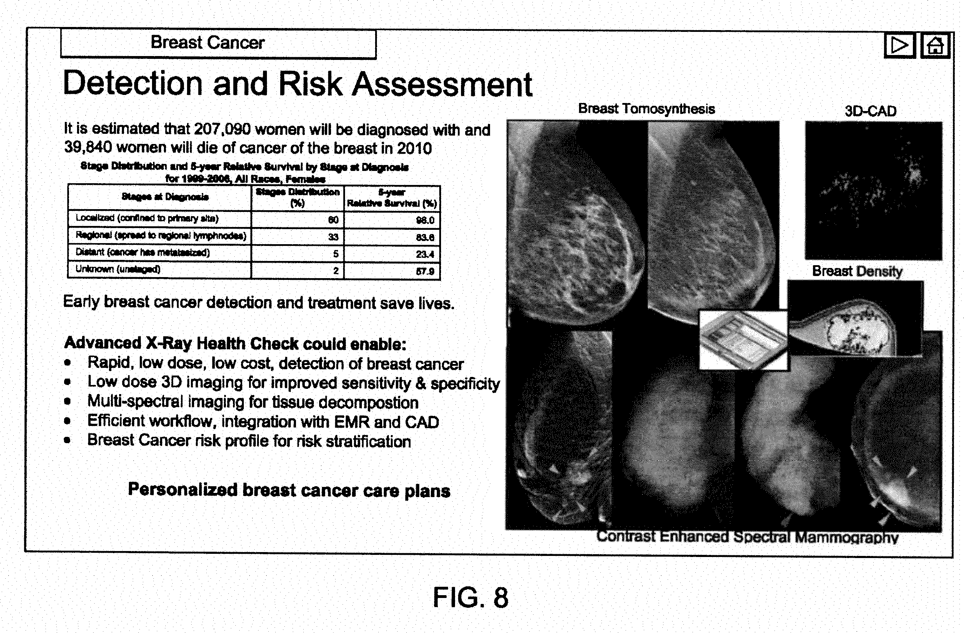

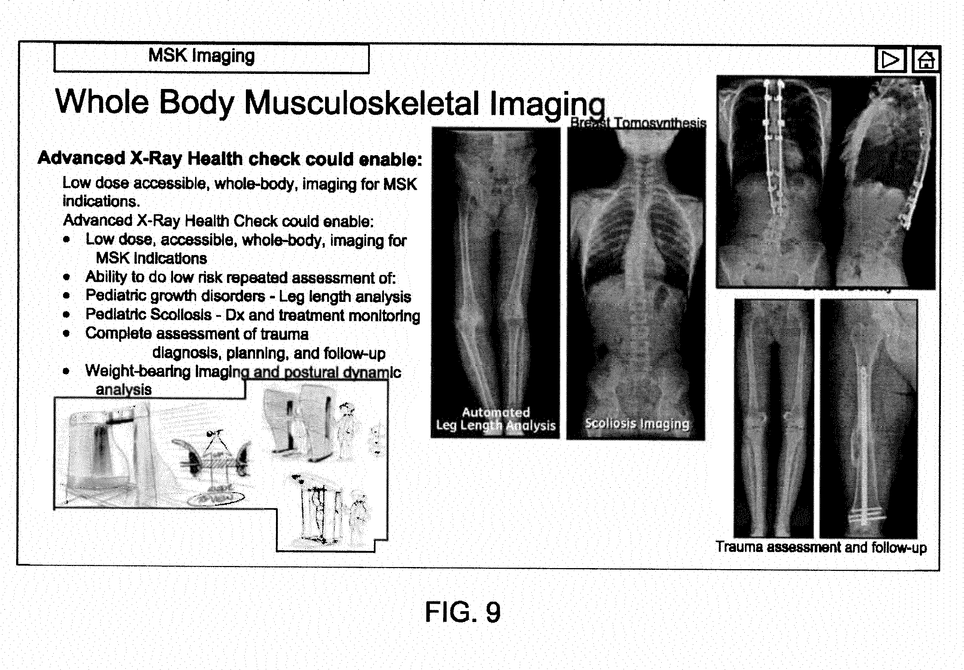

Referring now to FIGS. 4-9, clinical examples are provided of conditions and/or diseases for which diagnoses could be obtained by examination in health assessment system 10. FIG. 4 illustrates the detection and diagnosis of lung cancer by way of a subject examination in health assessment system 10, such as by utilizing multi-spectral 3D x-ray imaging with or without CAD and integration of the subject's EMR. FIG. 5 illustrates the detection and diagnosis of tuberculosis and/or another infectious disease by way of a subject examination in health assessment system 10, such as by utilizing x-ray imaging, integration of the subject's EMR, and a sputum test at the system 10. FIG. 6 illustrates the detection and diagnosis of cardiovascular disease by way of a subject examination in health assessment system 10, such as by utilizing dual energy radiography/x-ray imaging, calcium scoring, and a CAD risk assessment. FIG. 7 illustrates the detection and diagnosis of osteoarthritis by way of a subject examination in health assessment system 10, such as by 3D tomographic x-ray imaging, BMI analysis, and calculated morphometric measures and joint-space maps. FIG. 8 illustrates the detection and diagnosis of breast cancer by way of a subject examination in health assessment system 10, such as by utilizing multi-spectral 3D x-ray imaging, integration of the subject's EMR, and CAD risk profile for risk stratification. FIG. 9 illustrates the detection and diagnosis of musculoskeletal related disorders by way of a subject examination in health assessment system 10, such as by utilizing tomographic, weight bearing x-ray imaging, leg length analysis, postural dynamic analysis, and a CAD assessment. It is recognized that the clinical examples of conditions and/or diseases illustrated in FIGS. 4-9 are merely meant to be exemplary and that health assessment system 10 is capable of diagnosing/assessing other conditions and/or diseases for which a diagnosis can be obtained by examination of x-ray image data and other health related data.

Various embodiments of health assessment system 10 thus provide for a walk-through type health assessment system that acquires image data and other health related data that allow for the diagnosing or providing of health information to/about a subject. The health assessment system 10 enables the patient to operate the system independently, following written, audible or visual cues and prompts, without the assistance of trained medical personnel. In operation, the health assessment system 10 acquires health related data via subject interaction with a user interface 28 included thereon. The health assessment system 10 also acquires health related data upon the subject positioning himself/herself in the scanning area 18 of gate structure 12. That is, x-ray image data and other health related data are acquired by the x-ray source and detector combination 34, 36 and by the additional devices/sensors configured to acquire physiological and biological data, such as the electrical impedance percent body fat characterization device 47, the body thermal imaging device 48, weight measurement device (e.g., scale) 50, video capture device 52, and biomarker specimen collector 54, for example. Upon data acquisition, algorithms and/or programming on computer 42 enable automatic extraction of diagnostic information from the acquired data and provide a health related diagnosis or a recommendation for further action based thereon via the implementation of computer aided diagnosis (CAD).

Beneficially, health assessment system 10 thus functions to acquire and analyze a range of diagnostic test information so as to enable and facilitate a "one-stop" exam for a subject. Such a one-stop exam increases efficiency and lowers overall cost to a health care enterprise. By designing the system to be user-friendly and to operate independently of trained medical professionals, access to reliable health diagnoses is improved. The health assessment system 10 could be situated in many non-traditional locations either in areas where there is insufficient provider coverage (rural areas or developing countries), where more convenience is desired (shopping malls, drugstores), or where independent and rapid but comprehensive health screens are required (natural disasters, military deployments, border or facility control points).

According to one embodiment, the data acquisition process with subject positioned in the scanning area 18 takes a matter of seconds (e.g., 2 seconds), with the acquired image and health related data providing for diagnosing or providing health information to/about the subject. The short duration of the image acquisition by walk-through health assessment system 10 enables the subject to move through the device in an efficient and convenient manner, so as to provide high-throughput, efficient imaging of a large number of subjects in a very cost effective manner, if so required, and so as to minimizes the x-ray exposure experienced by the subject.

Therefore, according to one embodiment of the invention, a health assessment system includes a first support structure oriented in a vertical fashion, a second support structure oriented in a vertical fashion and spaced apart from the first support structure to define a scanning area configured to receive a subject to be scanned, an x-ray imaging system incorporated into the first and second support structures and configured to acquire x-ray image data from the subject in a standing position, and at least one additional data acquisition device configured to acquire health related data from the subject. The health assessment system also includes a computer programmed to receive the x-ray image data and the health related data and generate a diagnostic output based on the x-ray image data and the health related data, the diagnostic output comprising at least one of a health related diagnosis and a recommendation for future action.

According to another embodiment of the invention, a walk-through health assessment system includes a gate structure having a first vertical member and a second vertical member spaced apart from the first vertical member to defme a scanning area, an x-ray imaging system secured to the gate structure and configured to acquire x-ray image data from a subject when standing in the scanning area, and a means for acquiring additional health related data from the subject separate from the x-ray image data. The walk-through health assessment system also includes a computer programmed to receive the x-ray image data and the additional health related data, extract diagnostic information from the x-ray image data and the health related data, and generate a subject health assessment from the extracted diagnostic information.

According to yet another embodiment of the invention, a method of acquiring physiological and biological data on a subject by way of a walk-through health assessment system includes positioning a subject within an scanning area of a gate structure of the walk-through health assessment system, with the gate structure including a first vertical member and a second vertical member spaced apart from the first vertical to define a scanning area. The method also includes acquiring x-ray image data from the subject by way of a linear x-ray source and x-ray detector arrangement and acquiring additional health related data from the subject by way of at least one additional data acquisition device. The method further includes extracting diagnostic information from the x-ray image data and the additional health related data and generating a subject health assessment from the extracted diagnostic information.

This written description uses examples to disclose the invention, including the preferred mode, and also to enable any person skilled in the art to practice the invention, including making and using any devices or systems and performing any incorporated methods. The patentable scope of the invention is defined by the claims, and may include other examples that occur to those skilled in the art. Such other examples are intended to be within the scope of the claims if they have structural elements that do not differ from the literal language of the claims, or if they include equivalent structural elements with insubstantial differences from the literal languages of the claims.

Various aspects and embodiments of the present invention are defined by the following numbered clauses:
1. A health assessment system comprising:
   a first support structure oriented in a vertical fashion;
   a second support structure oriented in a vertical fashion and spaced apart from the first support structure to define a scanning area configured to receive a subject to be scanned;
   an x-ray imaging system incorporated into the first and second support structures and configured to acquire x-ray image data from the subject in a standing position;
   at least one additional data acquisition device configured to acquire health related data from the subject; and
   a computer programmed to:
      receive the x-ray image data and the health related data; and
      generate a diagnostic output based on the x-ray image data and the health related data, the diagnostic output comprising at least one of a health related diagnosis and a recommendation for future action.
2. The health assessment system of clause 1 wherein the x-ray imaging system comprises:
   a linear x-ray source affixed to the first support structure on one side of the scanning area and oriented in a vertical fashion, the linear x-ray source configured to emit x-rays towards the subject; and
   a linear detector arrangement affixed to the second support structure on another side of the scanning area and generally opposite the linear x-ray source and configured to receive x-rays after passing through the subject.
3. The health assessment system of any preceding clause wherein the linear x-ray source comprises one of a distributed x-ray source including an array of x-ray sources in a linear arrangement, a single stationary x-ray source having a linear vertical configuration, and a movable x-ray source configured to translate in a vertical direction.
4. The health assessment system of any preceding clause wherein the at least one additional data acquisition device comprises at least one of a body thermal imaging device, a video capture device, a biomarker specimen collector, and a weight measurement device; and
   wherein the health related data comprises at least one of basal temperature, bone density, height, weight, body mass index (BMI), body fat percentage, and derived metabolic health information.
5. The health assessment system of any preceding clause wherein the at least one additional data acquisition device comprises a user interface configured to receive an input from the subject of the health related data.
6. The health assessment system of any preceding clause wherein the user interface comprises a card reader configured to receive and read a subject specific health card having health related data stored thereon.
7. The health assessment system of any preceding clause wherein the user interface comprises a data input device configured to receive input from the subject regarding health related data, wherein the input comprises at least one of an identification number and health specific data.
8. The health assessment system of any preceding clause further comprising a communications device configured to access a remotely stored electronic medical record (EMR) of the subject, wherein the EMR can be retrieved responsive to the identification number input by the subject to the data input device.
9. The health assessment system of any preceding clause wherein the communications device is configured to transmit the diagnostic output to a remote location.
10. The health assessment system of any preceding clause wherein the user interface is further configured to generate prompts to the subject to assist in the acquisition of at least one of the x-ray image data and the health related data.
11. The health assessment system of any preceding clause wherein the x-ray image data comprises multi-spectral x-ray image data acquired from a dual energy x-ray scan.
12. The health assessment system of any preceding clause wherein the computer is further programmed to:
   control operation of the x-ray imaging system to acquire multiple projection images, the multiple projection images being acquired from an emission of x-rays at a plurality of vertically staggered locations; and
   generate a tomographic image of at least a portion of the subject from the multiple projection images.
13. A walk-through health assessment system comprising:
   a gate structure including a first vertical member and a second vertical member spaced apart from the first vertical member to define a scanning area;
   an x-ray imaging system secured to the gate structure and configured to acquire x-ray image data from a subject when standing in the scanning area;
   a means for acquiring additional health related data from the subject separate from the x-ray image data; and
   a computer programmed to:
      receive the x-ray image data and the additional health related data;
      extract diagnostic information from the x-ray image data and the health related data; and
      generate a subject health assessment from the extracted diagnostic information.
14. The walk-through health assessment system of any preceding clause wherein the x-ray imaging system comprises:
   a linear x-ray source positioned on one side of the scanning area and oriented in a vertical fashion, the linear x-ray source configured to emit x-rays towards the subject; and
   a linear detector arrangement positioned on another side of the scanning area and generally opposite the linear x-ray source and configured to receive x-rays after passing through the subject;
   wherein the linear x-ray source is configured to emit x-rays towards the subject from a plurality of locations relative to the linear detector arrangement so as to provide for acquisition of multiple projection images.
15. The walk-through health assessment system of any preceding clause wherein the means for acquiring additional health related data comprises at least one of a body thermal imaging device, a video capture device, a biomarker specimen collector, and a weight measurement device.
16. The walk-through health assessment system of any preceding clause wherein the means for acquiring additional health related data comprises a user interface configured to receive an input from the subject regarding additional health related data.
17. The walk-through health assessment system of any preceding clause wherein the user interface is further configured to generate prompts to the subject to assist in the acquisition of at least one of the x-ray image data and the additional health related data.
18. The walk-through health assessment system of any preceding clause further comprising a communications device configured to access a remotely stored electronic medical record (EMR) of the subject based on the subject input to the user interface, wherein the EMR is retrieved to provide the additional health related data.
19. A method of acquiring physiological and biological data on a subject by way of a walk-through health assessment system, the method comprising:
   positioning a subject within an scanning area of a gate structure of the walk-through health assessment system, the gate structure including a first vertical member and a second vertical member spaced apart from the first vertical to define a scanning area;
   acquiring x-ray image data from the subject by way of a linear x-ray source and x-ray detector arrangement;
   acquiring additional health related data from the subject by way of at least one additional data acquisition device;
   extracting diagnostic information from the x-ray image data and the additional health related data; and
   generating a subject health assessment from the extracted diagnostic information.
20. The method of any preceding clause wherein acquiring the additional health related data comprises measuring a physiological parameter of the subject by way of at least one sensing device.
21. The method of any preceding clause wherein acquiring the additional health related data comprises:
   prompting the subject to input the additional health related data; and
   receiving the additional health related data upon input thereof by the subject.
22. The method of any preceding clause wherein inputting the additional health related data comprises at least one of:
   swiping a card having health related data thereon through a card reader located on the gate structure;
   inputting specific health related data by way of a data input device located on the gate structure; and
   inputting a subject identification number by way of a data input device located on the gate structure.
23. The method of any preceding clause further comprising retrieving the additional health related data from a system separate from the walk-through health assessment system based on the identification number.
24. The method of any preceding clause wherein the additional health related data comprises health data included in an electronic medical record (EMR) of the subject.

## Claims

1. A health assessment system (10) comprising:
a first support structure (14) oriented in a vertical fashion;
a second support structure (16) oriented in a vertical fashion and spaced apart from the first support structure (14) to define a scanning area (18) configured to receive a subject to be scanned;
an x-ray imaging system (34, 36) incorporated into the first and second support structures (14, 16) and configured to acquire x-ray image data from the subject in a standing position;
at least one additional data acquisition device (28, 47, 48, 50, 52, 54) configured to acquire health related data from the subject; and
a computer (42) programmed to:
receive the x-ray image data and the health related data; and
generate a diagnostic output based on the x-ray image data and the health related data, the diagnostic output comprising at least one of a health related diagnosis and a recommendation for future action.

2. The health assessment system (10) of claim 1 wherein the x-ray imaging system (34, 36) comprises:
a linear x-ray source (34) affixed to the first support structure (14) on one side of the scanning area (18) and oriented in a vertical fashion, the linear x-ray source (34) configured to emit x-rays towards the subject; and
a linear detector arrangement (36) affixed to the second support structure (16) on another side of the scanning area (18) and generally opposite the linear x-ray source (34) and configured to receive x-rays after passing through the subject.

3. The health assessment system (10) of claim 2 wherein the linear x-ray source (34) comprises one of a distributed x-ray source including an array of x-ray sources in a linear arrangement, a single stationary x-ray source having a linear vertical configuration, and a movable x-ray source configured to translate in a vertical direction.

4. The health assessment system (10) of any preceding claim wherein the at least one additional data acquisition device (28, 47, 48, 50, 52, 54) comprises at least one of a body thermal imaging device (48), a video capture device(52), a biomarker specimen collector (54), and a weight measurement device (50); and
wherein the health related data comprises at least one of basal temperature, bone density, height, weight, body mass index (BMI), body fat percentage, and derived metabolic health information.

5. The health assessment system (10) of any preceding claim wherein the at least one additional data acquisition device comprises a user interface (28) configured to receive an input from the subject of the health related data.

6. The health assessment system (10) of claim 5 wherein the user interface (28) comprises a card reader (30) configured to receive and read a subject specific health card having health related data stored thereon.

7. The health assessment system (10) of claim 5 or claim 6 wherein the user interface (28) comprises a data input device (32) configured to receive input from the subject regarding health related data, wherein the input comprises at least one of an identification number and health specific data.

8. The health assessment system (10) of claim 7 further comprising a communications device (56) configured to access a remotely stored electronic medical record (EMR) of the subject, wherein the EMR can be retrieved responsive to the identification number input by the subject to the data input device (32).

9. The health assessment system (10) of any of claims 5 to 8 wherein the user interface (28) is further configured to generate prompts to the subject to assist in the acquisition of at least one of the x-ray image data and the health related data.

10. The health assessment system (10) of any preceding claim wherein the computer (42) is further programmed to:
control operation of the x-ray imaging system (34, 36) to acquire multiple projection images, the multiple projection images being acquired from an emission of x-rays at a plurality of vertically staggered locations; and
generate a tomographic image of at least a portion of the subject from the multiple projection images.
